# EUROPÄISCHE PATENTSCHRIFT

(11) **EP 0 718 002 B1**
(45) Veröffentlichungstag und Bekanntmachung des Hinweises auf die Patenterteilung: **23.12.1998**
(21) Anmeldenummer: 95118594.1
(22) Anmeldetag: 25.11.1995
(51) Int. Cl.: A61M 5/31

(54) **Zweikammer-Spritze und Verfahren zum Herstellen und Füller derselben**
Two compartment syringe and process for manufacture and filling thereof
Seringue à deux compartiments et procédé de fabrication et de remplissage

(30) Priorität: 22.12.1994 DE 4445969
(43) Veröffentlichungstag der Anmeldung: 26.06.1996
(73) Patentinhaber: Schott Glas, 55122 Mainz (DE); CARL-ZEISS-STIFTUNG trading as Schott Glas, 55122 Mainz (DE)
(72) Erfinder: Reinhard, Michael, Dr., D-55270 Ober-Olm (DE); Spallek, Michael, Dr., D-55218 Ingelheim (DE)
(74) Vertreter: Luderschmidt, Schüler & Partner GbR

(56) Entgegenhaltungen:
- EP-A- 0 520 618
- FR-A- 1 099 362
- GB-A- 2 010 681

## Beschreibung

Die Erfindung bezieht sich auf eine Zweikammer-Fertigspritze für zwei zu applizierende Komponenten gemäß dem Obergriff des Anspruchs 1.

Zweikammer-Fertigspritzen mit einem Spritzenzylinder der vorgenannten Art sind durch die DE 37 36 343 A1, DE 38 16 961 A1, EP 0 599 649 A1, EP 0 328 699 A1, DE 42 04 309 A1 und die Veröffentlichung von H. Vetter:
"Die Lyophilisierung von Arzneimitteln in Felrtigspritzen" in: Die Pharmazeutische Industrie, Jg. 46 (1984), Heft 10, S. 1045-1049 bekanntgeworden.

Zweikammer-Fertigspritzen werden in der Medizin zur Verabreichung von Präparaten eingesetzt, die aus zwei Komponenten bestehen. Es gibt dabei zwei Möglichkeiten, die Komponenten zu kombinieren, nämlich die Kombination flüssig/flüssig und die Kombination fest/flüssig.

Diese Zweikammer-Fertigspritzen haben den Vorteil, daß das Mischen der beiden Komponenten ohne Umfüllen in ein anderes Behältnis erfolgen kann und daß aus dem Behältnis heraus dann direkt die Verabreichung erfolgen kann. Die derzeit in der Praxis noch am weitesten verbreitete Vorgehensweise ist die, daß beispielsweise einer Ampulle mittels einer Kunststoff-Einmalspritze Wasser entnommen wird, dieses dann in ein Fläschchen mit Pulver überführt wird, dort das Pulver aufgelöst wird, die Lösung dann wieder in die Kunststoffspritze aufgezogen wird und erst dann verabreicht werden kann. Diese Handlingsschritte bedeuten einen großen Handhabungsaufwand und darüber hinaus besteht die Gefahr der Kontamination und Verwechslung der Präparate, weil die verwendeten Kunststoff-Einmalspritzen üblicherweise nicht zur Identifikation des Präparates gekennzeichnet sind.

Demgegenüber ist der Handhabungsaufwand bei den Zwei-Kammerspritzen wesentlich einfacher. Vor ihrer Aktivierung sitzt der Zwischenstopfen, auch Mittelstopfen genannt, auf der dem Spritzenkopf abgewandten Seite des Bypasses. Wird nun zwecks Applizieren die Kolbenstange am Injektionskolben angebracht und diese, unter Zuhilfenahme der Griffleiste, in üblicher Weise verschoben, dann bewegt er sich, aber auch der Zwischenkolben, in Richtung Spritzenkopf, über den gleichzeitig eine Entlüftung stattfindet. Erreicht der Zwischenstopfen den Bypass, dann kann die flüssige Komponente ihn umströmen, und die beiden Komponenten vermischen sich, bevor sie appliziert werden.

Bei den eingangs bezeichneten bekannten Zwei-Kammerfertigspritzen ist der Spritzenzylinder ein einstückiger Zylinderkörper aus Glas oder Kunststoff, der beide Kammern aufnimmt. Die beiden Komponenten müssen daher in ein- und denselben Körper gefüllt werden.

Damit ist jedoch mit Nachteil latent die Gefahr verbunden, daß die langzeitunverträglichen Komponenten als "Cross Contamination" in kleinen Mengen in die jeweils andere Kammer gelangen, was aus pharmazeutischer Sicht außerordentlich kritisch ist. Die Tatsache, daß zwei Kammern in einem einzigen Spritzenzylinder vereint sind, bringt auch insbesondere beim Lyophylisieren Nachteile. Zum Lyophylisieren muß der gesamte, relativ lange Spritzenzylinder in den Lyophylisator eingebracht werden, dessen Betrieb extrem aufwendig ist. Der Spritzenzylinderteil, der die zweite Kammer bildet, mußte funktionell nicht zwangsläufig mit in die Kammer. Es ergibt sich dadurch eine merklich schlechte Ausnutzung des Volumens im Lyophylisator, was wiederum beachtliche Auswirkungen auf die Kosten hat, die gerade bei Massenprodukten einen entscheidenden Einfluß auf die Wirtschaftlichkeit der Herstellung nehmen.

Aus der EP-A-520618 ist eine Fertigspritze der vorgenannten Art bekannt, die aus zwei stirnseitig über Flansch- oder Ringbundteil zusammengefügten Teilzylindern besteht.

Der Erfindung liegt die Aufgabe zugrunde, eine Zweikammerspritze zu ermöglichen, die von den Komponenten her kostengünstig herzustellen ist und bei der der Abfüllprozeß für die beiden Komponenten zur Vermeidung von "Cross Contamination" getrennt geführt werden kann und bei der - im Falle daß Lyophylisat als eine Komponente abgefüllt wird - der Prozeßraum des Lyophylisators optimal genutzt wird. Der Erfindung liegt ferner die Aufgabe zugrunde, ein entsprechendes kostengünstiges und eine "Cross Contamination" vermeidendes Verfahren zum Herstellen und Füllen der Zweikammerspritze anzugeben.

Die Lösung dieser Aufgabe gelingt durch die kennzeichnenden Merkmale des Anspruchs 1.

Hinsichtlich des Verfahrens zum Herstellen und Füllen der vollmontierten Fertigspritze gelingt die Lösung der Aufgabe dadurch, daß zunächst die beiden Teilzylinder des Spritzenkörpers hergestellt werden, danach die durch die Teilzylinder gebildeten Kammern gefüllt, ggf. lyophilisiert und verschlossen werden und die beiden Teilzylinder erst dann zusammengefügt werden.

Der Spritzenzylinder der erfindungsgemäßen Zweikammerspritze ist quasi die Zusammenfügung zweier, die Kammern vorgebenden Behältnisse, die eine getrennte Befüllung der Kammern erlauben, wie später noch erläutert wird, so daß eine Gefahr der "Cross Contamination" praktisch nicht besteht.

Ist eine Komponente ein Lyophylisat, dann braucht nur der zugeordnete Teilzylinder in den Lyophylisator eingebracht werden, was die Wirtschaftlichkeit des Herstellungsprozesses beachtlich erhöht.

Der Spritzenzylinder, d.h. der erste Teilzylinder, ist in üblicher Weise am vorderen Ende mit einem angeformten oder zweistuckig verbundenen (verschlossenen) Spritzenkopf versehen. Das andere, rückwärtige Ende des ersten Teilzylinders ist im montierten Zustand nach dem Befüllen mit dem Zwischenstopfen verschlossen. Aus dem ersten Teilzylinder kann daher ein geschlossenes Behältnis gebildet werden. Der zweite Teilzylinder ist dagegen im vormontierten Zustand zunächst nur an einem Ende mit dem Injektionskolben verschlossen, d.h., das durch den zweiten Teilzylinder vorgegebene Behältnis wird erst durch das Zusammenfügen der beiden Teilzylinder allseitig geschlossen.

Ist nach einer Ausgestaltung der Erfindung der Aufbau der Spritze so getroffen, daß der zweite Teilzylinder an dem der Fügeebene zugewandten Ende mittels eines zusätzlichen Hilfsstopfens ebenfalls verschlossen ist, kann man beide Behältnisse völlig getrennt befüllen und verschließen und als allseitig geschlossene Behältnisse zusammenfügen. Das gibt mehr Freiheitsgrade bei dem Befüllen und Montieren der Spritze. So kann z.B. in dem dem Spritzenkopf zugewandten ersten Teilzylinder ein fertiges Lyophylisat vorliegen, während in dem zweiten Teilzylinder, der mit der Griffleiste verbunden ist, Lösungsmittel vorliegt in der Form, daß es im Zylinder zwischen dem Kolbenstopfen und dem Hilfsstopfen eingeschlossen ist. Damit kann sogar eine sehr weitgehende Forderung erfüllt werden, nämlich die, daß das Lösungsmittel für die Zwei-Kammerspritze terminal sterilisiert ist.

Zwei-Kammerspritzen, bei denen nach einer Weiterbildung der Erfindung beide Teilzylinder des Spritzenzylinders aus Kunststoff bestehen, haben den Vorteil, daß diese Kunststoffteile sehr kostengünstig herzustellen, partikelarm zu halten und zu sterilisieren sind. Das zugehörige Verfahren sieht vor, daß die beiden Teilzylinder im Reinraum spritzgegossen, danach magaziniert in hermetischer Verpackung sterilisiert werden, die beiden durch die Teilzylinder gebildeten Kammern separat gefüllt, ggf. lyophilisiert und verschlossen werden, und die beiden Teilzylinder erst dann zusammengefügt werden.

Nach einer weiteren Ausgestaltung der Erfindung besteht der hintere Teilzylinder aus Glas. Diese Ausführung hat den Vorteil, daß die Werkstoffe für die Spritzenteilzylinder gezielt nach den entsprechenden Anforderungen ausgewählt werden können.

Bei Kunststoff-Teilzylindern lassen sich mit Vorteil die Barriereeigenschaften verbessern, wenn nach einer Ausgestaltung der Erfindung der Kunststoff-Teilzylinder innen oder außen beschichtet ist, wobei dem Fachmann entsprechende Schichtmaterialien aus metallischem, keramischem oder glasigem Material zur Verfügung stehen. Die Kunststoff-Teilzylinder können auch aus einer inneren und äußeren Schicht aus verschiedenen Kunststoffen bestehen, wobei zwischen den beiden Kunststoffschichten auch eine weitere Schicht aus metallischem, keramischem oder glasigem Material eingebracht sein kann. Der Kunststoff bzw. eine der Kunststoffschichten ist vorzugsweise ein zyklisches Olefin-Copolymer (COC), das glasig ist und eine geringe Wasserdampfdurchlässigkeit hat.

Eine derartige Ausführung hat den Vorteil, daß auch bei Komponenten mit höchsten Anforderungen an die Barriereeigenschaften des Packmittels Kunststoff verwendet werden kann. Der Kunststoff bildet als Strukturwerkstoff die entsprechende Form des Spritzenteilzylinders, während die Schicht die sperrende Funktion übernimmt.

Dem Fachmann stehen eine Reihe von bekannten Möglichkeiten zum Zusammenfügen beider Teilzylinder zur Verfügung. Es können stoffschlüssige (Schweißen) oder kraftsschlüssige (Kleben) Verbindungstechniken Verwendung finden. Ihre Eignung hängt grundsätzlich von den jeweils verwendeten Werkstoffen für die Spritzenteilzylinder ab. Sind beide Teilzylinder aus Kunststoff, kommt insbesondere eine Schweißverbindung in Frage; aber auch eine Verklebung ist möglich.

Gelangt beim Applizieren des Spritzeninhaltes auch der Injektionskolben in den Bypass im kopfseitigen Teilzylinder, dann ist der Spritzenzylinder rückwärtig "offen" und es kann, je nach Haltung der Spritze, eine Restflüssigkeit rückwärtig austreten. Um dies zu verhindern, ist die Fertigspritze nach der Erfindung so ausgebildet, daß an der Kolbenstange eine mit dem Innendurchmesser des Spritzenzylinders durchmessergleiche Rückhaltescheibe angebracht ist, derart, daß diese beim Applizieren in den Spritzenzylinder eintaucht, wenn auch der Injektionskolben in den Bypassbereich gelangt.

Es ist bekannt, den Bypass als angeformte Ausstülpung am Spritzenzylinder auszubilden.

Durch diese Ausstülpung wird verhindert, daß die Spritzenzylinder ideal dicht gepackt werden können. Deshalb werden solche Spritzenzylinder (aus Glas) üblicherweise nicht im gedrängten Zustand durch einen Heißluft-Sterilisationsofen gefahren, sondern müssen zuvor in eigens dafür gefertigte Vorrichtungen gestellt werden.

Dieser Nachteil entfällt, wenn gemäß der Erfindung der erste Teilzylinder aus Kunststoff besteht und an der inneren Wandung in axialer Richtung verlaufende, den Bypass bildende Vertiefungen aufweist.

Diese Ausgestaltung der Bypassnut als innere Ausnehmung der Wand, also als Wandstärkenreduzierung im Spritzenzylinder, ermöglicht es, daß die Spritzenzylinder ideal dicht bei der Verarbeitung gepackt werden können.

Weitere Ausgestaltungen und Vorteile der Erfindung ergeben sich aus der folgenden Beschreibung der in den Zeichnungen dargestellten Ausführungsbeispiele.

Es zeigen.
- Fig. 1: einen schematischen Längsschnitt durch eine Zweikammer-Fertigspritze mit dem erfindungsgemäßen, aus zwei Teilzylindern bestehenden Spritzenzylinder wobei zur Übersichtlichkeit, der Bypass als Ausstülpung nach dem Stand der Technik dargestellt ist,
- Fig. 2: eine Explosionsdarstellung der Teilzylinder des Spritzenzylinders nach Fig. 1 und deren Zusammenfügung mit einem Bypass nach dem stand der Technik,
- Fig. 3: eine Darstellung analog Fig. 2 jedoch mit einer anderen Ausgestaltung eines Teilzylinders,
- Fig. 4: die erfindungsgemäße Ausführungsform für den Bypass in dem frontseitigen Teilzylinder durch eine Vertiefung in der Zylinderwand,
- Fig. 5: zwei Alternativen zur Beschichtung der Kunststoffwand von Kunststoff-Teilzylinder,
- Fig. 6: ein Zusammenfügen der beiden Teilzylinder durch Verkleben,
- Fig. 7: ein Zusammenfügen durch Schweißen,
- Fig. 8: eine erste Ausführungsform des Spritzenkopfes,
- Fig. 9: eine zweite Ausführungsform des Spritzenkopfes.

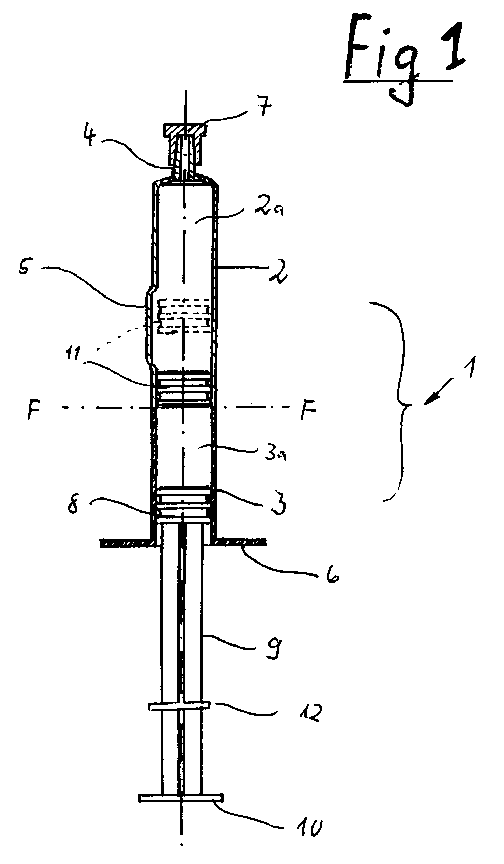

Die Fig. 1 zeigt in Form eines schematischen Längsschnittes den grundlegenden Aufbau der erfindungsgemäßen Fertigspritze für zwei zu applizierende Komponenten. Es können zwei flüssige Komponenten oder eine feste Komponente (Lyophylisat oder Pulver) und eine flüssige Komponente (Lösungsmittel) vorgesehen sein.

Die Zweikammer-Fertigspritze weist einen Spritzenzylinder 1 auf, der erfindungsgemäß aus zwei Teilzylindern 2 und 3 besteht, die in der Ebene F-F (Fügeebene), d.h. senkrecht zur Längsachse des Spritzenzylinders, zusammengefügt sind. Jeder Teilzylinder 2, 3 ist dabei einer Kammer 2a, 3a zugeordnet.

Am vorderen Ende des Spritzenzylinders 1, d.h. zugleich des Teilzylinders 2, ist ein Spritzenkopf 4 angeformt. Dieser Spritzenkopf kann grundsätzlich auch als getrenntes Bauteil zweistückig mit dem Spritzenzylinder bzw. zugleich seinem ersten Teilzylinder, verbunden sein. Ferner weist der vordere Teilzylinder 2 einen Bypas auf, hier zur Übersichtlichkeit in Form einer angeformten Ausstülpung 5 nach dem Stand der Technik. Eine übliche Griffleiste 6 ist zweistückig mit dem Spritzenzylinder, bzw. zugleich Teilzylinder am rückwartigen Ende verbunden. Auch hier ist, je nach dem für den Teilzylinder 3 verwendeten Werkstoff, eine Ausführung denkbar, bei der die Griffleiste 6 in das rückwärtige Ende des Teilzylinders eingesetzt bzw. eingeclipt wird. Derartige Ausführungen sind dem Fachmann geläufig, weil bekannt.

Der Spritzenzylinder 1 mit Spritzenkopf 4 und Griffleiste 6 bilden dabei den sogenannten Spritzenkörper. Der Spritzenkopf 4 ist durch eine übliche Verschlußkappe 7 - auch tip cap genannt - abgedeckt bzw. verschlossen. Im Bereich der Griffleiste 6 ist ein Injektionskolben 8 vorgesehen, der mittels einer Kolbenstange 9 mit Daumenauflage 10 im Spritzenzylinder 1 verschiebbar ist. Die beiden Kammern 2a, 3a sind durch einen Zwischenstopfen 11 (auch Mittelstopfen genannt) getrennt. Dieser Zwischenstopfen 11 ist dabei am rückwärtigen Ende des vorderen Teilzylinders 2 angeordnet und schließt die erste Kammer 2a gegen die zweite Kammer 3a auf Höhe der Fügeebene F-F ab.

An der Kolbenstange 9 ist eine mit dem Innendurchmesser des Spritzenzylinders durchmessergleiche Rückhaltescheibe 12 angebracht, ggf. angeformt, derart, daß diese beim Applizieren in den Spritzenzylinder eintaucht, sobald auch der Injektionskolben in den Bereich des Bypasses 5 gelangt. In dieser Stellung wäre der Spritzenzylinder rückwärtig praktisch "offen", und es könnte daher, je nach Haltung der Spritze, eine Restflüssigkeit rückwärtig austreten, was durch die Rückhaltescheibe 12 verhindert wird.

Bei der Ausführungsform nach Fig. 1 ist auf den Spritzenkopf 4 eine Verschlußkappe 7 aufgesetzt, die vor dem Applizieren abgenommen und durch eine Nadelhaube ersetzt wird.

Es sind dabei auch Ausführungen möglich, bei denen die Nadel unmittelbar in dem Spritzenkopf befestigt ist, beispielsweise mit dem Kunststoff des ersten, vorderen Teilzylinders 2, in einem Arbeitsvorgang umspritzt wird. Auch kann die Nadel in üblicher Weise in den Spritzenkopf eingeklebt sein. Wegen dieser Variationsmöglichkeiten ist unter dem Begriff "Fertigspritze" auch das bloße Behältnis ohne aufgesetzte bzw. eingebrachte Nadel zu verstehen.

Die in Fig. 1 dargestellte vollmontierte Fertigspritze mit dem zweigeteilten Spritzenzylinder wird in üblicher Weise beim Applizieren betätigt. Zunächst wird manuell durch Druck auf die Daumenauflage 10 der Kolbenstange 9 gegen die Fingerauflage 6 der Injektionskolben 8 in Richtung Spritzenkopf bewegt (über den gleichzeitig eine Entlüftung stattfindet). Dadurch bewegt sich auch der Zwischenkolben 11 in die gleiche Richtung und gibt, wenn er den Bypass 5 erreicht hat (gestrichelt dargestellt), die Verbindung zwischen den beiden Kammern 2a und 3a frei. In diesem aktivierten Zustand des Zwischenkolbens kann die Komponente der Kammer 2a, die im Regelfall eine flüssige Komponente ist, den Zwischenstopfen umströmen und mit der in der Kammer 2a befindlichen Komponente reagieren bzw. sich vermischen. Ist z.B. in der Kammer 2a ein Lyophylisat und in der Kammer 3a ein zugehöriges Lösungsmittel eingefüllt, dann wird das Lyophylisat in der aktivierten Stellung des Zwischenstopfens für die Applikation aufgelöst.

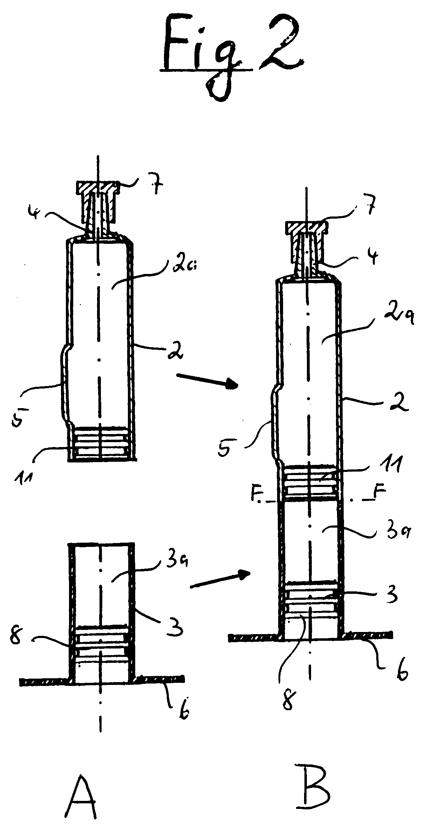

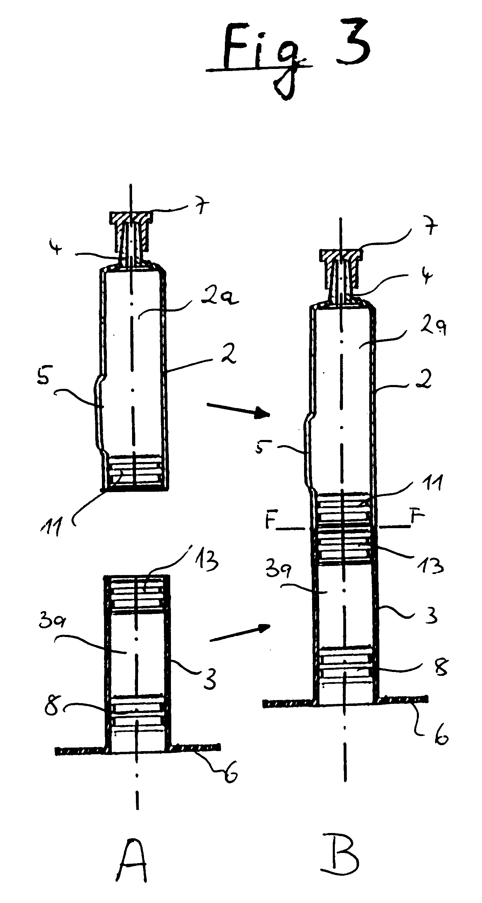

Der zweigeteilte Spritzenzylinder hat, wie bereits in der Beschreibungseinleitung grundsätzlich dargestellt, wesentliche Vorteile beim Herstellen und Befüllen des Spritzenkörpers, was nachfolgend anhand der Figuren 2 und 3 näher erläutert werden soll.

Die Fig. 2 zeigt im Abschnitt A die beiden Teilzylinder 2, 3 des Spritzenzylinders 1 der Fig. 1 im getrennten Zustand. Bei der Darstellung im Abschnitt B sind dann die beiden Teilzylinder - wie in Fig. 1 - in in der Fügeebene F-F zusammenmontiert.

Aus Fig. 2 wird deutlich, daß die erfindungsgemäße Doppelkammerspritze eine getrennte Befüllung der beiden Kammern erlaubt, so daß eine Gefahr der "Cross Contamination" praktisch nicht besteht. Erfordert nur eine Kammer eine besondere Behandlung bei dem Befüllen, z.B. wenn die entsprechende Komponente ein Lyophylisat ist, dann braucht nur der betreffende Teilzylinder und nicht der gesamte Spritzenzylinder behandelt zu werden, was die Wirtschaftlichkeit des Herstellungsprozesses maßgebend erhöht, insbesondere wenn besondere Behandlungsapparate notwendig sind, die eine dichte Stapelung im Behandlungsraum erfordern bzw. deren wirtschaftliche Betriebsweise maßgebend von der vollständigen Ausnutzung des Behandlungsraumes abhängt. Eine solche Anlage ist beispielsweise ein Lyophylisator.

Wie die Fig. 2, Abschnitt A, zeigt, ist der Teilzylinder 2 am vorderen Ende mit dem abgedeckten Spritzenkopf 4/7 verschlossen. Das rückwärtige Ende wird nach dem Befüllen der Kammer 2a mit dem Zwischenstopfen 11 verschlossen. Der Teilzylinder 2 bildet in diesem Zustand ein allseits geschlossenes Behältnis. Der Teilzylinder 3 ist am griffseitigen Ende mit dem Injektionskolben 8 verschlossen. Die Kammer 3a kann im dargestellten Zustand durch das offene, obere Ende des Teilzylinders befüllt werden. Danach wird, wie durch die Pfeile angedeutet, der Teilzylinder in der Fügeebene F-F auf den unteren Teilzylinder 3 aufgesetzt und mit diesem befestigt. Es ergibt sich dann der komplette Spritzenkörper gemäß dem Abschnitt B der Fig. 2.

Anhand der Fig. 2 wird deutlich, daß es mehrere Varianten zum Befüllen und Montieren der Teilzylinder gibt. So kann auch der Teilzylinder 3 ohne den Injektionskolben auf den allseits geschlossenen Teilzylinder 2 aufgesetzt und mit ihm montiert werden, danach - in umgekehrter Lage als in Fig. 2A dargestellt - befüllt und danach mit dem Injektionskolben 8 verschlossen werden. Entsprechend kann auch der Teilzylinder - separat oder mit dem Teilzylinder 3 zusammengefügt - vom Spritzenkopf her befüllt werden, wenn dies die Ausbildung des Spritzenkopfes zuläßt. Es ist auch eine Kombination der geschilderten Maßnahmen an den Teilzylindern im Rahmen des Zusammenfügens beider Teile möglich. Der Fachmann wird abhängig von dem konkreten Aufbau (Werkstoff) der Spritzenteile, dem verwendeten Herstellungsverfahren bzw. den einzubringenden Komponenten sich die fallspezifisch günstige Methode auswählen.

In der Fig. 3 ist eine weitere Ausführung des erfindungsgemäßen Spritzenkörpers nach Fig. 1 in der Explosionsdarstellungsart der Fig. 2 dargestellt. Gleiche Teile haben dieselben Bezugszeichen. Bei der Ausführung nach Fig. 3 ist das der Fügeebene F-F zugewandte, in den Fig. 1/2 noch offene Ende des Teilzylinders 3 nach dem Befüllen mittels eines zusätzlichen Hilfsstopfens 13 verschlossen. Bei dieser Ausführungsform kann man beide Teilzylinder - sprich Kammern - völlig getrennt Befüllen und Verschließen (Abschnitt A) und dann als allseits geschlossene Behältnisse - wie durch die Pfeile angedeutet - zum Spritzenkopf zusammenfügen (Abschnitt B).

Das Befüllen der Teilzylinder kann dabei, wie anhand der Fig. 2 erläutert, von beiden Enden der Teilzylinder aus in mehreren Kombinationsmöglichkeiten erfolgen.

Die Lösung nach Fig. 3 erlaubt mehr Freiheitsgrade beim Befüllen und Montieren der Spritze. So kann z.B. in der Kammer 2a ein fertiges, separat behandeltes Lyophylisat eingeschlossen sein, während in der Kammer 3a ein Lösungsmittel vollständig umschlossen ist. Dadurch ist es möglich, das Lösungsmittel für die Zweikammerspritze terminal zu sterilisieren.

Wie bereits in der Beschreibungseinleitung dargelegt, kann der hintere Teilzylinder entweder aus Glas oder aus Kunststoff bestehen.

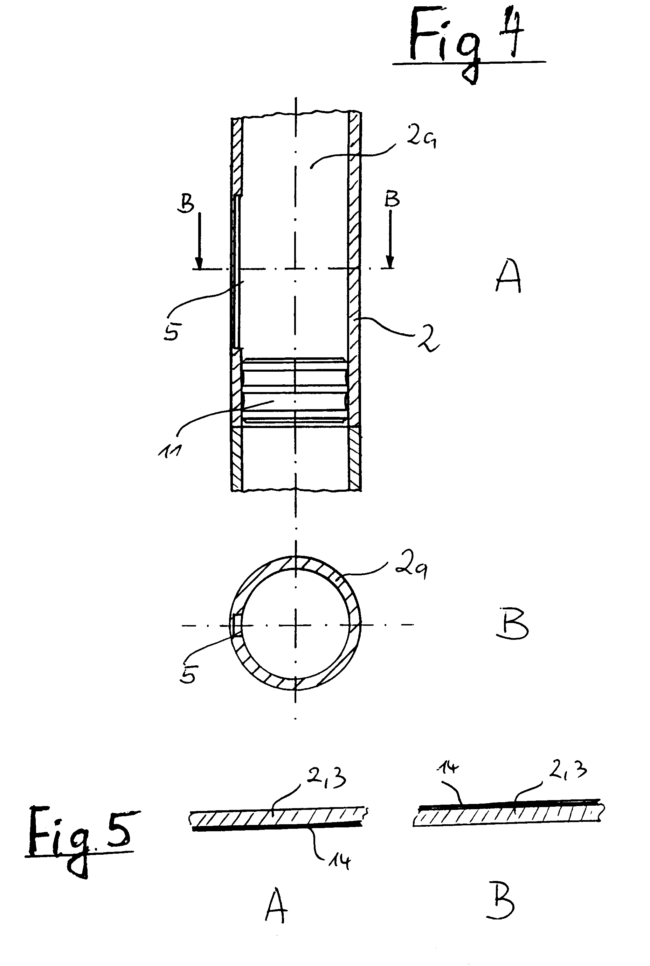

Die erfindungsgemäße Ausbildung des Bypasses 5 ist in Fig. 4 dargestellt.

Anstelle des in den Fig. 1-3 nach dem Stand der Technik dargestellten Bypasses in Form einer Ausstülpung wird gemäß Fig. 4 ein Bypass ohne Erweiterung des Zylinderdurchmessers vorgesehen. Ausstülpungen verhindern, daß die Spritzenzylinder ideal dicht gepackt werden können, und sie müssen bei der Behandlung in eigens dafür gefertigte Vorrichtungen gestellt werden. Diese Nachteile werden durch die Bypassausbildung nach Fig. 4 verhindert.

Die Fig. 4 zeigt im Abschnitt A einen Teillängsschnitt durch den vorderen Teilzylinder 2 mit eingesetztem Zwischenkolben 11. Im Abschnitt B ist ein Schnitt entlang der Linie B-B des Abschnittes A dargestellt. Die innere Wandung des Teilzylinders 2 weist im Bypassabschnitt mindestens eine in axialer Richtung verlaufende Vertiefung 5 (Rille, Nut etc.) auf, die den Bypass bildet.

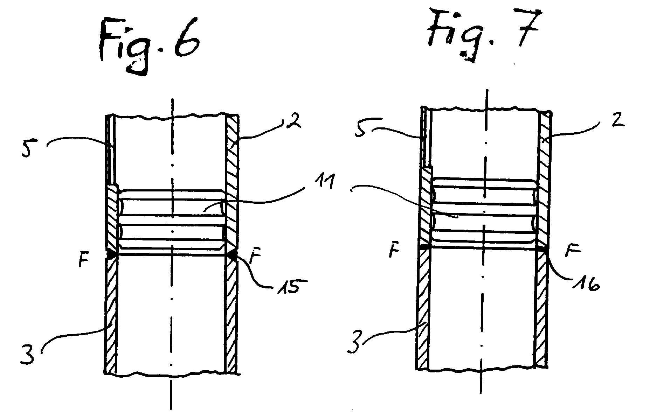

Wie bereits in der Beschreibungseinleitung erwähnt, sind die Kunststoff-Teilzylinder vorzugsweise innen oder außen beschichtet, um die Bartiereeigenschaften m verbessern. In Fig. 5 ist diese Beschichtung nochmals schematisch dargestellt. Die Wandung des Kunststoff-Teilzylinders 2 und/oder 3 ist innen (Abschnitt oder außen (Abschnitt B) mit einer Schicht 14 versehen. Es ist auch denkbar, innen und zugleich außen eine solche Schicht 14 auf die Zylinderwandung aufzubringen. Die Schicht kann aus metallischem, keramischem oder einem glasigen Material bestehen.

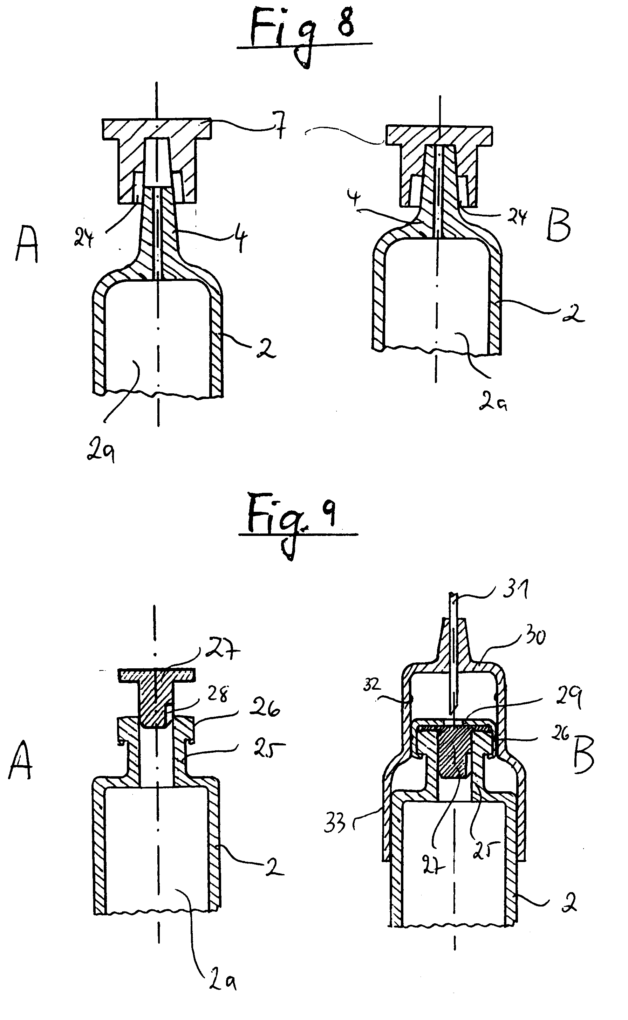

Zum Zusammenfügen beider Teilzylinder 2, 3 stehen dem Fachmann eine Reihe von Möglichkeiten zur Verfügung, die er fallspezifisch entsprechend den vorgegebenen Bedingungen und Gegebenheiten auswählen wird. Typische Verbindungen der beiden Teilzylinder sind in den Fig. 6 und 7 gezeigt.

Die Fig. 6 zeigt eine Ausführung, bei der die beiden Teilzylinder 2, 3 durch eine Verklebung in der Fügeebene F-F miteinander verbunden sind. Die Füge-Stirnseiten der beiden Teilzylinder sind dabei zwecks Ausbildung einer Klebenut 15 angefast. Diese Klebe-Verbindungstechnik kommt insbesondere dann in Betracht, wenn beide Teilzylinder aus Kunststoff bestehen.

Die Fig. 7 zeigt eine Ausführung, bei der beide Teilzylinder 2, 3 in der Fügeebene F-F durch eine Schweißnaht 16 miteinander verbunden sind. Diese Verbindungstechnik kann angewendet werden, wenn beide Teilzylinder aus Kunststoff bestehen.

Die Fig. 8 und 9 zeigen zwei Möglichkeiten zur Ausbildung und zum Verschließen des Spritzenkopfes, wobei das Verschließen jeweils in zwei Positioren A und B dargestellt ist. Die dargestellten Ausführungsformen kommen insbesondere in Betracht, wenn in der vorderen Kammer 2a ein Lyophylisat eingefüllt ist. Bei der Lyophilisietung muß nämlich eine Verbindung mit der Umgebung bestehen, damit die Dämpfe aus dem Spritzenzylinder entweichen können.

Die Fig. 8 zeigt - in vergrößerter Ansicht - den konisch zulaufenden Spritzenkopf 4 mit der Verschlußkappe (tip cap) 7. Diese Verschlußkappe weist axial verlaufende Ausnehmungen 24 auf, über die in der Stellung der Verschlußkappe gemaß dem Figurenabschnitt A, d.h. bei nicht völlig aufgesetzter Verschlußkappe 7, eine Verbindung der Kammer 2a des vorderen Teilzylinders 2 mit der Umgebung besteht.

Im Figurenabschnitt B ist die völlig aufgesetzte Verschlußkappe 7 dargestellt, d.h. das fertige Lyophylisat ist in der Kammer 2a eingeschlossen.

Bei der Ausführungsform nach Fig. 9 geht der Spritzenzylinder, d.h. der vordere Teilzylinder 2, in ein Mundstück 25 über, das eine im wesentlichen zylindrische Innenkontur und einen zu einem Außenbund 26 verstärkten Randwulst aufweist. In dieses Mundstück ist ein Stopfen 27 aus elastischem dichtenden Material mit einer Ausnehmung 28 eingesetzt, wobei der Figurenabschnitt A die Position des nicht völlig eingesetzten Stopfens zeigt. In dieser Stellung ist eine Verbindung zwischen der Umgebung und dem Inneren der Spritze gegeben.

Im Figurenabschnitt B ist der Stopfen völlig eingesetzt und mit einer Aluminiumkappe 29 umbördelt, die das fertige Lyophylisat dicht abschließt. Auf den Spritzenkopf ist eine Nadelhaube 30 aufgesetzt, in der eine Injektionsnadel 31 befestigt ist. Die Nadelhaube weist zwei Führungsabschnitte 32 und 33 auf, von denen der Abschnitt 32 zum umbördelten Außenbund und der Abschnitt 33 zum Teilzylinder 2 durchmessergleich ist. Diese Abschnitte dienen der Führung beim Aufschieben der Nadelhaube auf den Spritzenkörper, währenddessen die Nadel 31 den Stopfen 27 durchsticht und die Spritze für die Applikation bereit wird.

Ein bevorzugtes Verfahren zum Herstellen und Füllen der dargestellten Fertigspritzen besteht darin, daß zunächst die beiden Teilzylinder des Spritzenkörpers hergestellt werden, danach die durch die Teilzylinder gebildeten Kammern gefüllt, ggf. lyophilisiert und verschlossen werden und die beiden Teilzylinder erst dann zusammengefügt werden.

Besondere Vorteile bei der Herstellung bieten sich an, wenn beide Teilzylinder aus Kunststoff bestehen, die im Reinraum spritzgegossen werden, danach magaziniert in hermetischer Verpackung sterilisiert werden, und, wenn anschließend die beiden durch die Teilzylinder gebildeten Kammern separat gefüllt, ggf. lyophilisiert sowie verschlossen, die beiden Teilzylinder erst dann zusammengefügt werden.

Bei einer derartigen Herstellung werden teure Waschvorgänge vermieden und es ist eine einfache Sterilisation durch eine Gammastrahlung, alternativ durch Ethylenoxidgas, möglich. Verwendet man dabei moderne Kunststoffe wie ein zyklisches Olefin-Copolymer (COC), dann erhält man einen glasig transparenten Spritzenkörper mit hervorragenden Eigenschaften.

## Patentansprüche

1. Zweikammer-Fertigspritze für zwei zu applizierende Komponenten, mit einem Spritzenzylinder (1), der aus zwei stirnseitig zusammengefügten, jeweils eine Kammer bildenden Teilzylindern (2,3) besteht,
- von denen der erste Teilzylinder (2) aus Kunstoff besteht und ein oder zweistückig mit einem Spritzenkopf (4) verbunden ist und einen Bypaß (5) aufweist, und der an dem dem Spritzenkopf entgegengesetzten Ende mit einem die beiden Kammern abtrennenden Zwischenstopfen (11) verschlossen ist, und
- von denen der zweite Teilzylinder (3) am der Fügestelle abgewandten Ende eine Griffleiste (6) aufweist und mit einem Injektionskolben (8) verschlossen ist, an dem eine Kolbenstange (9) befestigbar ist,
dadurch gekennzeichnet, daß
- der erste Teilzylinder (2) an der inneren Wandung in axialer Richtung verlaufende, den Bypaß ohne Erweiterung des Zylinderdurchmessers bildende Vertiefungen (5) aufweist,
- die beiden Teilzylinder (2, 3) an der Fügestelle (F) bündig zusammengefügt sind, und die Griffleiste (6) zweistückig mit dem zweiten Teilzylinder (3) verbunden ist.

2. Fertigspritze nach Anspruch 1, dadurch gekennzeichnet daß der zweite Teilzylinder (3) an dem der Fügeebene (F) zugewandten Ende mittels eines zusätzliche Hilfsstopfens (13) verschlossen ist.

3. Fertigspritze nach Anspruch 1 oder 2, dadurch gekennzeichnet, daß die beiden Teilzylinder (2, 3) aus Kunststoff bestehen.

4. Fertigspritze nach Anspruch 1 oder 2, dadurch gekennzeichnet, daß der hintere Teilzylinder (2, 3) aus Glas besteht.

5. Fertigspritze nach Anspruch 3 oder 4, dadurch gekennzeichnet, daß mindestens ein Kunststoff-Teilzylinder innen und/oder außen beschichtet ist.

6. Fertigspritze nach Anspruch 3, dadurch gekennzeichnet, daß die beiden Teilzylinder (2, 3) durch eine Verschweißung miteinander verbunden sind.

7. Fertigspritze nach einem der Ansprüche 3-5, dadurch gekennzeichnet, daß die beiden Teilzylinder (2, 3) durch eine Verklebung miteinander verbunden sind.

8. Fertigspritze nach Anspruch 7, dadurch gekennzeichnet, daß die Füge-Stirnseiten der Teilzylinder (2, 3) zwecks Ausbildung einer Klebenut (15) angefast sind.

9. Fertigspritze nach mindestens einem der Ansprüche 1-8, dadurch gekennzeichnet, daß an der Kolbenstange (9) eine mit dem Innendurchmesser des Spritzenzylinders (1) durchmessergleiche Rückhaltescheibe (12) angebracht ist, derart, daß diese beim Applizieren in den Spritzenzylinder eintaucht, wenn auch der Injektionskolben (8) in den Bypaßbereich gelangt.

10. Verfahren zum Herstellen und Füllen der Fertigspritze nach Anspruch 1 oder einem der folgenden, dadurch gekennzeichnet, daß zunächst die beiden Teilzylinder des Spritzenkörpers hergestellt werden, danach die durch die Teilzylinder gebildeten Kammern gefüllt, gegebenenfalls lyophilisiert und verschlossen werden und die beiden Teilzylinder erst dann zusammengefügt werden.

11. Verfahren nach Anspruch 10 für eine Fertigspritze nach Anspruch 3, dadurch gekennzeichnet, daß die beiden Kunststoff-Teilzylinder im Reinraum spritzgegossen, danach magaziniert in hermetischer Verpackung sterilisiert werden, die beiden durch die Teilzylinder gebildeten Kammern separat gefüllt, gegebenenfalls lyophilisiert und verschlossen werden, und die beiden Teilzylinder erst dann zusammengefügt werden.

## Claims

1. Two-compartment ready-to-use syringe for two components to be applied, having a syringe cylinder (1) which comprises two part cylinders (2, 3) joined together at the ends and respectively forming a compartment,
- of which the first part cylinder (2) consists of plastic and is connected in one or two pieces to a syringe head (4) and has a bypass (5), and which is connected at the end opposite the syringe head to an intermediate stopper (11), dividing the two compartments, and
- of which the second part cylinder (3) has at the end facing away from the joint a gripper bar (6) and is closed by an injection piston (8), on which a piston rod (9) can be fastened,
characterized in that
- the first part cylinder (2) has, on the inner walling, depressions (5) running in the axial direction and forming the bypass without increasing the diameter of the cylinder,
- the two part cylinders (2, 3) are joined flush together at the joint (F), and
- the gripper bar (6) is connected in two pieces to the second part cylinder (3).

2. Ready-to-use syringe according to Claim 1, characterized in that the second part cylinder (3) is closed at the end facing the joining plane (F) by means of an additional auxiliary stopper (13).

3. Ready-to-use syringe according to Claim 1 or 2, characterized in that the two part cylinders (2, 3) consist of plastic.

4. Ready-to-use syringe according to Claim 1 or 2, characterized in that the rear part cylinder (2, 3) consists of glass.

5. Ready-to-use syringe according to Claim 3 or 4, characterized in that at least one plastic part cylinder is coated on the inside and/or outside.

6. Ready-to-use syringe according to Claim 3, characterized in that the two part cylinders (2, 3) are connected to each other by a weld.

7. Ready-to-use syringe according to one of Claims 3 - 5, characterized in that the two part cylinders (2, 3) are connected to each other by an adhesive bond.

8. Ready-to-use syringe according to Claim 7, characterized in that the joining end faces of the part cylinders (2, 3) are bevelled for the purpose of forming an adhesive groove (15).

9. Ready-to-use syringe according to at least one of Claims 1-8, characterized in that a retaining disc (12), of the same diameter as the inside diameter of the syringe cylinder (1), is attached to the piston rod (9) in such a way that the said retaining disc enters the syringe cylinder during application whenever the injection piston (8) enters the bypass region.

10. Process for manufacturing and filling the ready-to-use syringe according to Claim 1 or one of the following claims, characterized in that firstly the two part cylinders of the syringe body are manufactured, after which the compartments formed by the part cylinders are filled, if required are lyophilized and closed, and the two part cylinders are only then joined together.

11. Process according to Claim 10 for a ready-to-use syringe according to Claim 3, characterized in that the two plastic part cylinders are injection-moulded in a clean room, are then sterilized in a magazined form in hermetic packaging, the two compartments formed by the part cylinders are separately filled, if required are lyophilized and closed, and the two part cylinders are only then joined together.

## Revendications

1. Seringue complète à deux compartiments pour deux composants à appliquer, avec un cylindre de seringue (1), qui se compose de deux cylindres partiels (2, 3) formant chacun un compartiment et assemblés bout à bout,
- dont le premier cylindre partiel (2) est constitué de matière synthétique et est assemblé en une ou en deux pièces avec une tête de seringue (4) et présente un bipasse (5), et qui est obturé, à l'extrémité opposée à la tête de seringue, avec un tampon intermédiaire (11) séparant les deux compartiments, et
- dont le second cylindre partiel (3) présente une patte de saisie (6) à son extrémité située à l'opposé de la zone d'assemblage et est obturé avec un piston d'injection (8), auquel peut se fixer une tige de piston (9),
caractérisée en ce que
- le premier cylindre partiel (2) présente à la paroi intérieure des bossages (5) orientés en direction axiale, formant le bipasse sans élargissement du diamètre du cylindre,
- les deux cylindres partiels (2, 3) sont assemblés à fleur à la zone d' assemblage (F), et
- la patte de saisie (6) est assemblée en deux pièces avec le second cylindre partiel (3)

2. Seringue complète suivant la revendication 1, caractérisée en ce que le second cylindre partiel (3) est obturé avec un tampon auxiliaire supplémentaire (13) à son extrémité tournée vers le plan d'assemblage (F).

3. Seringue complète suivant la revendication 1 ou 2, caractérisée en ce que les deux cylindres partiels (2, 3) sont constitués de matière synthétique.

4. Seringue complète suivant la revendication 1 ou 2, caractérisée en ce que le cylindre partiel arrière (2, 3) est constitué de verre.

5. Seringue complète suivant la revendication 3 ou 4, caractérisée en ce qu'au moins un cylindre partiel en matière synthétique est revêtu intérieurement et/ou extérieurement.

6. Seringue complète suivant la revendication 3, caractérisée en ce que les deux cylindres partiels (2, 3) sont assemblés l'un à l'autre par une soudure.

7. Seringue complète suivant l'une quelconque des revendications 3 - 5, caractérisée en ce que les deux cylindres partiels (2, 3) sont assemblés l'un à l'autre par un collage.

8. Seringue complète suivant la revendication 7, caractérisée en ce que les faces extrêmes d'assemblage des cylindres partiels (2, 3) sont chanfreinées pour former une rainure de collage (15).

9. Seringue complète suivant au moins l'une des revendications 1 - 8, caractérisée en ce qu'un disque de retenue (12), ayant le même diamètre que le diamètre intérieur du cylindre de seringue (1), est monté sur la tige de piston (9), d'une façon telle que, lors de l'application, il plonge dans le cylindre de seringue, lorsque le piston d'injection (8) arrive aussi dans la zone du bipasse.

10. Procédé pour la fabrication et le remplissage de la seringue complète suivant la revendication 1 ou l'une des suivantes, caractérisé en ce que l'on fabrique d'abord les deux cylindres partiels du corps de seringue, on remplit ensuite les compartiments formés par les cylindres partiels, le cas échéant on les lyophilise et on les obture, et l'on assemble seulement alors les deux cylindres partiels.

11. Procédé suivant la revendication 10 pour une seringue complète suivant la revendication 3, caractérisé en ce que l'on moule par injection les deux cylindres partiels en matière synthétique dans une chambre blanche, ensuite on les emmagasine et on les stérilise dans un emballage hermétique, on remplit séparément les deux compartiments formés par les cylindres partiels, le cas échéant on les lyophilise et on les obture, et on assemble seulement alors les deux cylindres partiels.
